Europäisches Patentamt
European Patent Office
Office européen des brevets

(19)

(11) Veröffentlichungsnummer: **0 273 262**
**A2**

(12) # EUROPÄISCHE PATENTANMELDUNG

(21) Anmeldenummer: **87118275.4**

(22) Anmeldetag: **10.12.87**

(51) Int. Cl.⁴: **C07D 405/04**, A61K 31/44, A61K 31/405

(30) Priorität: **23.12.86 DE 3644094**
**07.08.87 DE 3726261**

(43) Veröffentlichungstag der Anmeldung:
**06.07.88 Patentblatt 88/27**

(84) Benannte Vertragsstaaten:
**AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Häusler, Günther, Prof. Dr.**
**Albert-Schweitzer Strasse 13**
**D-6104 Seeheim(DE)**
Erfinder: **Gericke, Rolf, Dr.**
**Mozartstrasse 19**
**D-6104 Seeheim(DE)**
Erfinder: **Wurziger, Hanns, Dr.**
**Greinstrasse 7B**
**D-6100 Darmstadt(DE)**
Erfinder: **Baumgarth, Manfred, Dr.**
**Sachsenstrasse 53**
**D-6100 Darmstadt(DE)**
Erfinder: **Lues, Inge, Dr.**
**Paul-Wagner-Strasse 53**
**D-6100 Darmstadt(DE)**
Erfinder: **De Peyer, Jacques, Dr.**
**Bleichweg 15**
**D-6104 Seeheim(DE)**
Erfinder: **Bergmann, Rolf, Dr.**
**Birkenhag 36**
**D-6101 Reichelsheim(DE)**

(54) **Chromanderivate.**

(57) Chromanderivate der Formel I

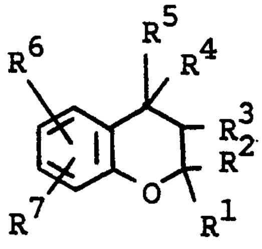

I

worin $R^1$ bis $R^7$ die in Patentanspruch 1 angegebenen Bedeutungen haben, zeigen Wirkungen auf das cardiovaskuläre System.

**EP 0 273 262 A2**

## Chromanderivate

Die Erfindung betrifft neue Chromanderivate der Formel I

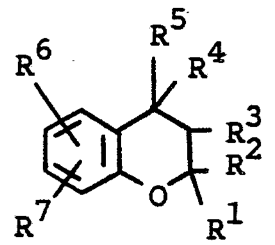

I

worin

$R^1$ A,
$R^2$ H oder A,
$R^1$ und $R^2$ zusammen auch Alkylen mit 3-6 C-Atomen,
$R^3$ OH oder OAc,
$R^4$ H,
$R^3$ und $R^4$ zusammen auch eine Bindung,

$R^5$ einen unsubstituierten oder ein-oder zweifach durch A, F, C1, Br, J, OH, OA, OAc, $NO_2$, $NH_2$, AcNH, HOOC und/oder AOOC substituierten 1H-2-Pyridon-1-yl-, 1H-6-Pyridazinon-1-yl, 1H-2-Pyrimidinon-1-yl, 1H-6-Pyrimidinon-1-yl, 1H-2-Pyrazinon-1-yl oder 1H-2-Thiopyridon-1-yl-rest, wobei diese Reste auch partiell hydriert sein können,

$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$ $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C(=$NNH_2$),

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten,

sowie deren Salze.

Ähnliche Verbindungen sind bekannt aus der EP-A1-76075 und der EP-A1-173848.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z. B. Widerstandsabnahme und Flußzunahme auf, wobei der Einfluß auf die Herzfrequenz geringt bleibt. Weiterhin zeigen die Verbindungen eine relaxierende Wirkung auf verschiedene glattmuskuläre Organe (Gastrointestinaltrakt, Respirationssystem und Uterus). Die Wirkungen der Verbindungen können mit Hilfe an sich bekannter Methoden ermittelt werden, wie sie z. B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z. B. Mäuse, Ratten, Meerschweinchen, Hunde, Katzen, Affen oder Schweine.

Die Verbindungen können daher als Arzneimittelwirkstoffe in der Human-und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls $R^1$ und $R^2$ zusammen Alkylen bedeuten, so ist die Alkylengruppe vorzugsweise unverzweigt, im einzelnen bevorzugt $-(CH_2)_n-$, wobei n 3, 4, 5 oder 6 bedeutet.

Die Gruppe "alkyl" steht vorzugsweise für $-CH_2-$oder $-CH_2CH_2-$.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m-oder p-Toluyl, 1-oder 2-Naphthoyl.

$R^1$ und $R^2$ sind vorzugsweise jeweils Alkyl, insbesondere jeweils Methyl oder Ethyl, bevorzugt jeweils Methyl.

$R^3$ und $R^4$ sind bevorzugt zusammen eine Bindung. Falls $R^4$ H bedeutet, ist $R^3$ bevorzugt OH, O-CHO oder $O-COCH_3$.

$R^5$ ist bevorzugt unsubstituiertes 1H-2-Pyridon-1-yl, 1H-2-Pyrazinon-1-yl oder 1H-4-Hydroxy-2-pyridon-1-yl, ferner bevorzugt unsubstituiertes 1H-6-Pyridazinon-1-yl, 4,5-Dihydro-1H-6-pyridazinon-1-yl, 1H-2-Pyrimidinon-1-yl, 1H-6-Pyrimidinon-1-yl oder 1H-2-Thiopyridon-1-yl. Falls $R^5$ einen substituierten Pyridon- bzw. Thiopyridonring bedeutet, so ist dieser Ring vorzugsweise einfach in 3-, 4-oder 5-Stellung oder zweifach in 3-und 5-Stellung substituiert. Besonders bevorzugte Substituenten sind OH, $NO_2$ und $NH_2$, ferner AOOC, OA, Cl, Br und $NHCOCH_3$, besonders bevorzugte substituierte Reste $R^5$ im einzelnen 4-, ferner 3-, 5-und 6-Hydroxy-, 3-, 4-, 5-oder 6-Methoxy-, 3-, 4-, 5-oder 6-Acetoxy-, 3-, 5-oder 6-Chlor-, 3-oder 5-Nitro-, 3-oder 5-Amino-, 3-oder 5-Carboxy-, 3-oder 5-Methoxycarbonyl-, 3-oder 5-Ethoxycarbonyl-, 3-oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido-und 3-Acetamido-5-brom-1H-2-pyridon-1-yl bzw. -1H-2-thiopyridon-1-yl, 1H-3-, 1H-4-oder 1H-5-Hydroxy-6-pyridazinon-1-yl, 1H-3-, 1H-4-oder 1H-5-Ethoxycarbonyl-6-pyridazinon-1-yl, 1H-4-, 1H-5-oder 1H-6-Hydroxy-2-pyrimidinon-1-yl, 1H-2-oder 1H-4-Hydroxy-6-pyrimidinon-1-yl.

$R^5$ kann ferner bevorzugt bedeuten: 3,4-Dihydro-1H-2-pyridon-1-yl, 2,3-Dihydro-6H-2-pyridon-1-yl, 5,6-Dihydro-1H-2-pyridon-1-yl, 2,3-Dihydro-1H-6-pyridazinon-1-yl, 1,2-Dihydro-5H-6-pyridazinon-1-yl, 3,4-Dihydro-1H-2-pyrimidinon-1-yl, 1,6-Dihydro-3H-2-pyrimidinon-1-yl, 5,6-Dihydro-1H-2-pyrimidinon-1-yl, 2,3-Dihydro-1H-6-pyrimidinon-1-yl, 1,2-Dihydro-5H-6-pyrimidinon-1-yl, 4,5-Dihydro-1H-6-pyrimidinon-1-yl, 3,4-Dihydro-1H-2-pyrazinon-1-yl, 1,6-Dihydro-3H-2-pyrazinon-1-yl, 5,6-Dihydro-1H-2-pyrazinon-1-yl, 3,4-Dihydro-1H-2-thiopyridon-1-yl, 2,3-Dihydro-1H-2-thiopyridon-1-yl, 5,6-Dihydro-1H-2-thiopyridon-1-yl.

In $R^6$ und $R^7$ bedeuten vorzugsweise:

A: Methyl, ferner Ethyl;
AO: Methoxy, ferner Ethoxy;
ACO: Acetyl, ferner Propionyl;
ACS: Thioacetyl, ferner Thiopropionyl;
AOOC: Methoxycarbonyl, ferner Ethoxycarbonyl;
AO-CS: Methoxy-thiocarbonyl, ferner Ethoxy-thiocarbonyl;
ACOO: Acetoxy, ferner Propionoxy;
ACSO: Thio(no)acetoxy, ferner Thio(no)propionoxy;
Hydroxylakyl: Hydroxymethyl oder 1-oder 2-Hydroxyethyl;
Mercaptoalkyl: Mercaptomethyl oder 1-oder 2-Mercaptoethyl;
NHA: Methylamino, ferner Ethylamino;
$NA_2$: Dimethylamino, ferner Diethylamino;
ASO: Methylsulfinyl, ferner Ethylsulfinyl;
$ASO_2$: Methylsulfonyl, ferner Ethylsulfonyl;
AO-SO: Methoxy-sulfinyl, ferner Ethoxy-sulfinyl;
$AO-SO_2$: Methoxy-sulfonyl, ferner Ethoxy-sulfonyl;
Ac-NH: Acetamido, ferner Formamido, Propionamido oder Benzamido;
AO-CO-NH: Methoxycarbonylamino, ferner Ethoxycarbonylamino;
HANSO: Methylaminosulfinyl, ferner Ethylaminosulfinyl;

$A_2NSO$: Dimethylaminosulfinyl, ferner Diethylaminosulfinyl;
$HANSO_2$: Methylaminosulfonyl, ferner Ethylaminosulfonyl;
$A_2NSO_2$: Dimethylaminosulfonyl, ferner Diethylaminosulfonyl;
HANCO: N-Methylcarbamoyl, ferner N-Ethylcarbamoyl;
$A_2NOC$: N,N-Dimethylcarbamoyl, ferner N,N-Diethylcarbamoyl;
HANCS: N-Methyl-thiocarbamoyl, ferner N-Ethyl-thiocarbamoyl;
$A_2NCS$: N,N-Dimethyl-thiocarbamoyl, ferner N,N-Diethyl-thiocarbamoyl;
ASONH: Methylsulfinylamino, ferner Ethylsulfinylamino;
$ASO_2NH$: Methylsulfonylamino, ferner Ethylsulfonylamino;
AOSONH: Methoxysulfinylamino, ferner Ethoxysulfinylamino;
$AOSO_2NH$: Methoxysulfonylamino, ferner Ethoxysulfonylamino;
ACO-alkyl: 2-Oxopropyl, 2-Oxobutyl, 3-Oxobutyl, 3-Oxopentyl;
Nitroalkyl: Nitromethyl, 1-oder 2-Nitroethyl;
Cyanalkyl: Cyanmethyl, 1-oder 2-Cyanethyl;
A-C(= NOH): 1-Oximinoethyl, ferner 1-Oximinopropyl;
A-C(= $NNH_2$): 1-Hydrazonoethyl, ferner 1-Hydrazonopropyl.

Die Reste $R^6$ und $R^7$ stehen vorzugsweise in 6-und 7-Stellung des Chromansystems. Sie können jedoch auch in 5-und 6-, 5-und 7-, 5-und 8-, 6-und 8-sowie 7-und 8-Stellung stehen.

Von den Resten $R^6$ und $R^7$ ist vorzugsweise der eine H, während der andere von H verschieden ist. Dieser andere Rest steht vorzugsweise in 6-Stellung, aber auch in 5-, 7-oder 8-Stellung, und ist vorzugsweise CN oder $NO_2$, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, $CF_3$, $H_2NCO$, $H_2NCS$ oder $NH_2$.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch

in Ia $R^1$ und $R^2$ jeweils A bedeuten;

in Ib $R^1$ und $R^2$ jeweils $CH_3$ bedeuten;

in Ic $R^1$ und $R^2$ zusammen Alkylen mit 3-6 C-Atomen bedeuten;

in Id $R^5$ 1H-2-Pyridon-1-yl, 1H-2-Pyrazinon-1-yl, 1H-6-Pyridazinon-1-yl, 4,5-Dihydro-1H-6-pyridazinon-1-yl, 1H-2-Pyrimidinon-1-yl, 1H-6-Pyrimidinon-1-yl, 1H-2-Thiopyridon-1-yl, 3-, 4-, 5-oder 6-Hydroxy-, 3-, 4-, 5-oder 6-Methoxy-, 3-, 4-, 5-oder 6-Acetoxy-, 3-, 5-oder 6-Chlor-, 3-oder 5-Nitro-, 3-oder 5-Amino-, 3-oder 5-Carboxy-, 3-oder 5-Methoxycarbonyl-, 3-oder 5-Ethoxycarbonyl-, 3-oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-, 3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamido-oder 3-Acetamido-5-brom-1H-2-pyridon-1-yl oder -1H-2-thiopyridon-1-yl, 1H-3-, 1H-4-oder 1H-5-Hydroxy-6-pyridazinon-1-yl, 1H-3-, 1H-4-oder 1H-5-Ethoxycarbonyl-6-pyridazinon-1-yl, 1H-4-, 1H-5-oder 1H-6-Hydroxy-2-pyrimidinon-1-yl, 1H-2-oder 1H-4-Hydroxy-6-pyrimidinon-1-yl bedeutet;

in Ie $R^5$ 1H-2-Pyridon-1-yl, 1H-2-Pyrazinon-1-yl oder 1H-4-Hydroxy-2-pyridon-1-yl bedeutet;

In If $R^5$ 1H-2-Pyridon-1-yl bedeutet;

in Ig $R^1$ und $R^2$ jeweils $CH_3$ und

$R^5$ 1H-2-Pyridon-1-yl, 1H-2-Pyrazinon-1-yl, 1H-6-Pyridazinon-1-yl, 4,5-Dihydro-1H-6-pyridazinon-1-yl, 1H-2-Pyrimidinon-1-yl, 1H-6-Pyrimidinon-1-yl, 1H-2-Thiopyridon-1-yl, 3-, 4-, 5-oder 6-Hydroxy-, 3-, 4-, 5-oder 6-Methoxy-, 3-, 4-, 5-oder 6-Acetoxy-, 3-, 5-oder 6-Chlor-, 3-oder 5-Nitro-, 3-oder 5-Amino-, 3-oder 5-Carboxy-, 3-oder 5-Methoxycarbonyl-, 3-oder 5-Ethoxycarbonyl-, 3-oder 5-Acetamido-, 3,5-Dichlor-, 3,5-Dibrom-, 3-Chlor-5-nitro-, 3-Nitro-5-chlor-, 3-Brom-5-nitro-, 3-Nitro-5-brom-, 3,5-Dinitro-,3-Chlor-5-amino-, 3-Amino-5-chlor-, 3-Brom-5-amino-, 3-Amino-5-brom-, 3-Chlor-5-acetamido-, 3-Acetamido-5-chlor-, 3-Brom-5-acetamidooder 3-Acetamido-5-brom-1H-2-pyridon-1-yl oder -1H-2-thiopyridon-1-yl, 1H-3-, 1H-4-oder 1H-5-Hydroxy-6-pyridazinon-1-yl, 1H-3-, 1H-4-oder 1H-5-Ethoxycarbonyl-6-pyridazinon-1-yl, 1H-4-, 1H-5-oder IH-6-Hydroxy-2-pyrimidinon-1-yl, 1H-2-oder 1H-4-Hydroxy-6-pyrimidinon-1-yl bedeuten;

in Ih $R^1$ und $R^2$ jeweils $CH_3$ und

$R^5$ 1H-2-Pyridon-1-yl, 1H-2-Pyrazinon-1-yl oder 1H-4-Hydroxy-2-pyridon-1-yl bedeuten;

in Ii $R^1$ und $R^2$ jeweils $CH_3$ und
$R^5$ 1H-2-Pyridon-1-yl bedeuten.

Weiterhin sind bevorzugt Verbindungen der Formeln I′ sowie Ia′ bis Ii′, die den Formeln I sowie Ia bis Ii entsprechen, worin jedoch jeweils zusätzlich $R^3$ OH, OCHO oder $OCOCH_3$ und $R^4$ H bedeuten.

Weiterhin sind bevorzugt Verbindungen der Formeln I″ sowie Ia″ bis Ii″, die den Formeln I sowie Ia bis Ii entsprechen, worin jedoch jeweils zusätzlich $R^3$ und $R^4$ zusammen eine Bindung bedeuten.

Ferner sind bevorzugt Verbindungen der Formeln I, I′, I″, Ia bis Ii, Ia′ bis Ii′ sowie Ia″ bis Ii″, worin jeweils zusätzlich

(a) $R^6$ von H verschieden ist und
$R^7$ H bedeutet;
(b) $R^6$ von H verschieden ist und in 6-Stellung steht und
$R^7$ H bedeutet;
(c) $R^6$ $NO_2$, CN, CHO, ACO, HOOC, AOOC, ACOO, F, Cl, Br, J, $CF_3$, $H_2NCO$, $H_2NCS$ oder $NH_2$ und
$R^7$ H bedeutet;
(d) $R^6$ $NO_2$, CN, CHO, ACO, HOOC, AOOC, ACOO, F, Cl, Br, J, $CF_3$, $H_2NCO$, $H_2NCS$ oder $NH_2$ bedeutet und in 6-Stellung steht und
$R^7$ H bedeutet;
(e) $R^6$ $NO_2$, CN, CHO, $CH_3CO$, $CH_3OOC$,$C_2H_5OOC$ oder $CH_3COO$ und
$R^7$ H bedeutet;
(f) $R^6$ $NO_2$, CN, CHO, $CH_3CO$, $CH_3OOC$,$C_2H_5OOC$ oder $CH_3COO$ bedeutet und in 6-Stellung steht und
$R^7$ H bedeutet;
(g) $R^6$ $NO_2$ oder CN und
$R^7$ H bedeutet;
(h) $R^6$ $NO_2$ oder CN bedeutet und in 6-Stellung steht und
$R^7$ H bedeutet;
(i) $R^6$ CN und
$R^7$ H bedeutet;
(j) $R^6$ CN bedeutet und in 6-Stellung steht und
$R^7$ H bedeutet.

Im übrigen haben vor-und nachstehend die Reste $R^1$ bis $R^7$, A, "alkyl" und Ac die bei Formel I angegebenen Bedeutungen, wenn nicht ausdrücklich etwas anderes angegeben ist.

Gegenstand der Erfindung ist ferner ein Verfahren zur Herstellung der Verbindungen der Formel I sowie ihrer Salze, das dadurch gekennzeichnet ist, daß man ein 3,4-Epoxychroman der Formel II

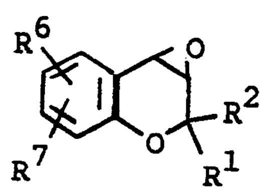

II

worin

$R^1$, $R^2$, $R^6$ und $R^7$ die bei Formel I angegebene Bedeutung haben,

mit einer Verbindung der Formel III

$R^5$-H III

worin $R^5$ die bei Formel I angegebene Bedeutung hat oder mit einem ihrer reaktionsfähigen Derivate

umsetzt

und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden die Verbindungen der Formel I durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Die Ausgangsstoffe II und III sind in der Regel bekannt. Sofern sie nicht bekannt sind, können sie nach an sich bekannten Methoden hergestellt werden. So sind die Ausgangsstoffe der Formel II erhältlich durch Umsetzung von Propargylchloriden der Formel $HC\equiv C^1\text{-}CR^1R^2\text{-}Cl$ mit Phenolen der Formel $R^6R^7C_6H_3OH$ zu Phenolethern der Formel $R^6R^7C_6H_3OCR^1R^2\text{-}C\equiv CH$, Cyclisierung zu Chromenen entsprechend Formel I, worin $R^3$ und $R^4$ zusammen eine Bindung bedeuten, aber an Stelle des Restes $R^5$ ein H-Atom steht, Anlagerung von HOBr zum Bromhydrin (IV) entsprechend Formel I, $R^3$ = OH, aber Br an Stelle von $R^4$ und H an Stelle von $R^5$, und Dehydrobromierung (Methode vgl. z. B. EP-A1-76075).

Als reaktionsfähige Derivate von III eignen sich die entsprechenden Salze, z. B. die Na-oder K-Salze, die auch in situ entstehen können.

Es ist zweckmäßig, in Gegenwart einer Base zu arbeiten. Als Basen eignen sich z. B. Alkalimetall-oder Erdalkalimetall-hydroxide, -hydride oder auch -amide wie NaOH, KOH, $Ca(OH)_2$, NaH, KH, $CaH_2$, $NaNH_2$, $KNH_2$, ferner organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran oder Dioxan; Glykolether wie Ethylenglykolmonomethyl-oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II kann auch in situ hergestellt werden, z. B. durch Einwirkung einer Base auf das entsprechende Bromhydrin IV.

Eine Verbindung der Formel I, worin $R^3$ = OH und $R^4$ = H ist, kann durch Behandeln mit einem Dehydratisierungsmittel in eine Verbindung der Formel I, worin $R^3$ und $R^4$ zusammen eine Bindung bedeuten, umgewandelt werden. Das gelingt z. B. durch Einwirkung einer der angegebenen Basen, z. B. NaH, in einem der angegebenen Lösungsmittel, z. B. DMSO, bei Temperaturen zwischen 0 und 150 °.

Weiterhin kann man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandeln.

Beispielsweise ist es möglich, daß man ein H-Atom mittels einer Halogenierung durch ein Halogenatom oder einer Nitrierung durch eine Nitrogruppe ersetzt und/oder eine Nitrogruppe zu einer Aminogruppe reduziert und/oder eine Amino-oder Hydroxygruppe alkyliert oder acyliert und/oder eine Cyangruppe (z.B. mit HCl in Wasser/Methanol bei 20-100°) in eine Carboxylgruppe oder (z. B. mit Raney-Nickel in Wasser/Essigsäure/Pyridin in Gegenwart von Natriumphosphat) in eine Formylgruppe oder (z. B. mit KOH in tert.-Butanol) in eine Carbamoylgruppe oder (z. B. mit $H_2S$ in Pyridin/Triethylamin) in eine Thiocarbamoylgruppe umwandelt und/oder einen substituierten oder unsubstituierten 1H-2-Pyridon-1-ylrest (z. B. mit $P_2S_5$ oder mit Lawesson-Reagenz in Toluol) in den entsprechendne 1H-2-Thiopyridon-1-ylrest umwandelt.

Eine Nitrierung gelingt unter üblichen Bedingungen, z. B. mit einem Gemisch aus konzentrierter $HNO_3$ und konzentrierter $H_2SO_4$ bei Temperaturen zwischen 0 und 30 °. Falls mindestens einer der Substituenten

$R^6$ und $R^7$ eine elektronegative Gruppe wie CN oder $NO_2$ bedeutet, erfolgt die Nitrierung überwiegend am Rest $R^5$; andernfalls erhält man in der Regel Gemische, bei denen die Nitrogruppen am Rest $R^5$ oder am Chromanring stehen können.

Analoges gilt für die Halogenierung, die z. B. mit elementarem Chlor oder Brom in einem der üblichen inerten Lösungsmittel bei Temperaturen zwischen etwa 0 und 30 ° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z. B. Verbindungen der Formeln A-Cl, A-Br oder A-J oder entsprechende Schwefelsäure-oder Sulfonsäureester wie Methylchlorid, -bromid, -jodid, Dimethylsulfat, Methyl-p-toluolsulfonat. Ferner kann man z. B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z. B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino-oder Hydroxygruppen eignen sich zweckmäßig die Halogenide (z. B. Chloride oder Bromide) oder Anhydride von Carbonsäuren der Formel Ac-OH, z. B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z. B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0 ° und etwa 160 °, vorzugsweise zwischen 20 ° und 120 °. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z. B. Verbindungen der Formel I, worin $R^1 = R^2$, $R^3$ = OH und $R^4$ = H ist, zwei chirale Zentren; bei der Herstellung durch Reaktion von II mit III entsteht jedoch ganz überwiegend nur ein Racemat mit trans-Stellung der Substituenten $R^3$ = OH und $R^5$. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z. B. optisch aktive Säuren, wie die D- und L-Formen von Weinsäure, Dibenzoylweinsäure, Diacetylweinsäure, Camphersulfonsäuren, Mandelsäure, Äpfelsäure oder Milchsäure. Carbinole (I, $R^3$ = OH) können ferner mit Hilfe chiraler Acylierungsreagenzien, z. B. D-oder L-α-Methylbenzylisocyanat, verestert und dann getrennt werden (vgl. EP-A1-120428). Die verschiedenen Formen der Diastereomeren können in an sich bekannter Weise, z. B. durch fraktionierte Kristallisation, getrennt, und die Enantiomeren der Formel I können in an sich bekannter Weise aus den Diastereomeren in Freiheit gesetzt werden. Enantiomerentrennungen glingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-und Geschmacks-und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, peripheren oder cerebralen Gefäßerkrankungen, sowie Krankheitszuständen, die mit Bluthochdruck verbunden sind.

Dabei werden die erfindungsgemäßen Substanzen in der Regel in Analogie zu bekannten Antianginosa bwz. Blutdrucksenkern, z. B. Nicorandil oder BRL-34915 [2,2-Dimethyl-4-(2-oxo-1-pyrrolidinyl)-6-cyan-chroman-3-ol; vgl. EP-A1-173848] verabreicht, vorzugsweise in Dosierungen zwischen etwa 0,1 und 50 mg, insbesondere zwischen 0,2 und 5 mg pro Dosierungseinheit. Die tägliche Dosierung liegt vorzugsweise zwischen etwa 0,001 und 1, insbesondere zwischen 0,003 und 0,1 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachfolgenden Beispielen bedeutet "übliche Aufarbeitung":

Man gibt, falls erforderlich, Wasser hinzu, extrahiert mit einem organischen Lösungsmitte wie Ethylacetat, trennt ab, trocknet die organische Phase über Natriumsulfat, filtriert, dampft ein und reinigt durch Chromatographie und/oder Kristallisation.

Vor-und nachstehend sind alle Temperaturen in °C angegeben.

## Beispiel 1

Ein Gemisch von 20,1 g 2,2-Dimethyl-3,4-epoxy-6-cyanchroman ("IIa"), 9,5 g 1H-2-Pyridon ("Pyridon"), 3 g einer 80 %igen Dispersion von NaH in Paraffinöl und 600 ml DMSO wird 16 Std. bei 20 ° gerührt, in Wasser gegossen und mit Ethylacetat extrahiert. Man dampft ein und chromatographiert den Rückstand über Kieselgel. Mit Dichlormethan wird 2,2-Dimethyl-4-(1H-2-pyridon-1-yl)-6-cyan-2H-chromen ("A"; F. 146-148 °) eluiert, anschliessend mit Ethylacetat 2,2-Dimethyl-4-(1H-2-pyridon-1-yl)-6-cyan-chroman-3-ol ("B"; F. 244 °); Mengenverhältnis "A":"B" etwa 9:7.

Analog erhält man

2,2-Dimethyl-4-(1H-2-thiopyridon-1-yl)-6-cyan-2H-chromen
2,2-Dimethyl-4-(1H-2-thiopyridon-1-yl)-6-cyan-chroman-3-ol
2,2-Dimethyl-4-(1H-3-chlor-2-pyridon-1-yl)-6-cyan-2H-chromen
2,2-Dimethyl-4-(1H-3-chlor-2-pyridon-1-yl)-6-cyan-chroman-3-ol
2,2-Dimethyl-4-(1H-5-chlor-2-pyridon-1-yl)-6-cyan-2H-chromen, F. 185-188°
2,2-Dimethyl-4-(1H-5-chlor-2-pyridon-1-yl)-6-cyan-chroman-3-ol, F. 268-270°
2,2-Dimethyl-4-(1H-6-chlor-2-pyridon-1-yl)-6-cyan-2H-chromen
2,2-Dimethyl-4-(1H-6-chlor-2-pyridon-1-yl)-6-cyan-chroman-3-ol
2,2-Dimethyl-4-(1H-3-hydroxy-2-pyridon-1-yl)-6-cyan-2H-chromen

2,2-Dimethyl-4-(1H-3-hydroxy-2-pyridon-1-yl)-6-cyan-chroman-3-ol, F. 262-265°
2,2-Dimethyl-4-(1H-4-hydroxy-2-pyridon-1-yl)-6-cyan-2H-chromen, F. 290-295°
2,2-Dimethyl-4-(1H-4-hydroxy-2-pyridon-1-yl)-6-cyan-chroman-3-ol, F. 248-250°
2,2-Dimethyl-4-(1H-5-hydroxy-2-pyridon-1-yl)-6-cyan-2H-chromen
2,2-Dimethyl-4-(1H-5-hydroxy-2-pyridon-1-yl)-6-cyan-chroman-3-ol, F. 256,5-258°
2,2-Dimethyl-4-(1H-3-methoxy-2-pyridon-1-yl)-6-cyan-2H-chromen
2,2-Dimethyl-4-(1H-3-methoxy-2-pyridon-1-yl)-6-cyan-chroman-3-ol, F. 245-248°
2,2-Dimethyl-4-(1H-3-acetoxy-2-pyridon-1-yl)-6-cyan-2H-chromen
2,2-Dimethyl-4-(1H-3-acetoxy-2-pyridon-1-yl)-6-cyan-chroman-3-ol, F. 260-263°
2,2-Dimethyl-4-(1H-3-nitro-2-pyridon-1-yl)-6-cyan-2H-chromen, F. 148-150°
2,2-Dimethyl-4-(1H-3-nitro-2-pyridon-1-yl)-6-cyan-chroman-3-ol, F. 240-242°
2,2-Dimethyl-4-(1H-5-nitro-2-pyridon-1-yl)-6-cyan-2H-chromen, F. 214-216°
2,2-Dimethyl-4-(1H-5-nitro-2-pyridon-1-yl)-6-cyan-chroman-3-ol, F. 249-251°
2,2-Dimethyl-4-(1H-3-amino-2-pyridon-1-yl)-6-cyan-2H-chromen
2,2-Dimethyl-4-(1H-3-amino-2-pyridon-1-yl)-6-cyan-chroman-3-ol, F. 213-215°
2,2-Dimethyl-4-(1H-5-amino-2-pyridon-1-yl)-6-cyan-2H-chromen, F. 180°
2,2-Dimethyl-4-(1H-5-amino-2-pyridon-1-yl)-6-cyan-chroman-3-ol, F. 260-264°
2,2-Dimethyl-4-(1H-3-acetamido-2-pyridon-1-yl)-6-cyan-2H-chromen
2,2-Dimethyl-4-(1H-3-acetamido-2-pyridon-1-yl)-6-cyan-chroman-3-ol, F. 274-276°
2,2-Dimethyl-4-(1H-5-acetamido-2-pyridon-1-yl)-6-cyan-2H-chromen, F. 255-256°
2,2-Dimethyl-4-(1H-5-acetamido-2-pyridon-1-yl)-6-cyan-chroman-3-ol, F. 303-305°
2,2-Dimethyl-4-(1H-3-carboxy-2-pyridon-1-yl)-6-cyan-2H-chromen
2,2-Dimethyl-4-(1H-3-carboxy-2-pyridon-1-yl)-6-cyan-chroman-3-ol, F. 250-253°
2,2-Dimethyl-4-(1H-5-carboxy-2-pyridon-1-yl)-6-cyan-2H-chromen
2,2-Dimethyl-4-(1H-5-carboxy-2-pyridon-1-yl)-6-cyan-chroman-3-ol, F. 238-240°
2,2-Dimethyl-4-(1H-3,5-dichlor-2-pyridon-1-yl)-6-cyan-2H-chromen, F. 230-232°
2,2-Dimethyl-4-(1H-3,5-dichlor-2-pyridon-1-yl)-6-cyan-chroman-3-ol, F. 167-170°
2,2-Dimethyl-4-(1H-3,5-dibrom-2-pyridon-1-yl)-6-cyan-2H-chromen, F. 268-270°
2,2-Dimethyl-4-(1H-3,5-dibrom-2-pyridon-1-yl)-6-cyan-chroman-3-ol, F. 207-209°
2,2-Dimethyl-4-(1H-3-chlor-5-nitro-2-pyridon-1-yl)-6-cyan-2H-chromen
2,2-Dimethyl-4-(1H-3-chlor-5-nitro-2-pyridon-1-yl)-6-cyan-chroman-3-ol
2,2-Dimethyl-4-(1H-3-nitro-5-chlor-2-pyridon-1-yl)-6-cyan-2H-chromen
2,2-Dimethyl-4-(1H-3-nitro-5-chlor-2-pyridon-1-yl)-6-cyan-chroman-3-ol
2,2-Dimethyl-4-(1H-3-brom-5-nitro-2-pyridon-1-yl)-6-cyan-2H-chromen
2,2-Dimethyl-4-(1H-3-brom-5-nitro-2-pyridon-1-yl)-6-cyan-chroman-3-ol
2,2-Dimethyl-4-(1H-3-nitro-5-brom-2-pyridon-1-yl)-6-cyan-2H-chromen
2,2-Dimethyl-4-(1H-3-nitro-5-brom-2-pyridon-1-yl)-6-cyan-chroman-3-ol
2,2-Dimethyl-4-(1H-3,5-dinitro-2-pyridon-1-yl)-6-cyan-2H-chromen
2,2-Dimethyl-4-(1H-3,5-dinitro-2-pyridon-1-yl)-6-cyan-chroman-3-ol
2,2-Dimethyl-4-(1H-3-chlor-5-amino-2-pyridon-1-yl)-6-cyan-2H-chromen
2,2-Dimethyl-4-(1H-3-chlor-5-amino-2-pyridon-1-yl)-6-cyan-chroman-3-ol
2,2-Dimethyl-4-(1H-3-amino-5-chlor-2-pyridon-1-yl)-6-cyan-2H-chromen
2,2-Dimethyl-4-(1H-3-amino-5-chlor-2-pyridon-1-yl)-6-cyan-chroman-3-ol
2,2-Dimethyl-4-(1H-3-brom-5-amino-2-pyridon-1-yl)-6-cyan-2H-chromen
2,2-Dimethyl-4-(1H-3-brom-5-amino-2-pyridon-1-yl)-6-cyan-chroman-3-ol
2,2-Dimethyl-4-(1H-3-amino-5-brom-2-pyridon-1-yl)-6-cyan-2H-chromen
2,2-Dimethyl-4-(1H-3-amino-5-brom-2-pyridon-1-yl)-6-cyan-chroman-3-ol
2,2-Dimethyl-4-(1H-3-chlor-5-acetamido-2-pyridon-1-yl)-6-cyan-2H-chromen
2,2-Dimethyl-4-(1H-3-chlor-5-acetamido-2-pyridon-1-yl)-6-cyan-chroman-3-ol
2,2-Dimethyl-4-(1H-3-acetamido-5-chlor-2-pyridon-1-yl)-6-cyan-2H-chromen
2,2-Dimethyl-4-(1H-3-acetamido-5-chlor-2-pyridon-1-yl)-6-cyan-chroman-3-ol
2,2-Dimethyl-4-(1H-3-brom-5-acetamido-2-pyridon-1-yl)-6-cyan-2H-chromen
2,2-Dimethyl-4-(1H-3-brom-5-acetamido-2-pyridon-1-yl)-6-cyan-chroman-3-ol
2,2-Dimethyl-4-(1H-3-acetamido-5-brom-2-pyridon-1-yl)-6-cyan-2H-chromen
2,2-Dimethyl-4-(1H-3-acetamido-5-brom-2-pyridon-1-yl)-6-cyan-chroman-3-ol
2,2-Dimethyl-4-(1H-2-pyridon-1-yl)-6-nitro-2H-chromen,
F. 158°
2,2-Dimethyl-4-(1H-2-pyridon-1-yl)-6-nitro-chroman-3-ol, F. 229-231°

2,2-Dimethyl-4-(1H-2-thiopyridon-1-yl)-6-nitro-2H-chromen
2,2-Dimethyl-4-(1H-2-thiopyridon-1-yl)-6-nitro-chroman-3-ol
2,2-Dimethyl-4-(1H-3-chlor-2-pyridon-1-yl)-6-nitro-2H-chromen
2,2-Dimethyl-4-(1H-3-chlor-2-pyridon-1-yl)-6-nitro-chroman-3-ol
2,2-Dimethyl-4-(1H-5-chlor-2-pyridon-1-yl)-6-nitro-2H-chromen
2,2-Dimethyl-4-(1H-5-chlor-2-pyridon-1-yl)-6-nitro-chroman-3-ol
2,2-Dimethyl-4-(1H-6-chlor-2-pyridon-1-yl)-6-nitro-2H-chromen
2,2-Dimethyl-4-(1H-6-chlor-2-pyridon-1-yl)-6-nitro-chroman-3-ol
2,2-Dimethyl-4-(1H-3-hydroxy-2-pyridon-1-yl)-6-nitro-2H-chromen
2,2-Dimethyl-4-(1H-3-hydroxy-2-pyridon-1-yl)-6-nitro-chroman-3-ol
2,2-Dimethyl-4-(1H-4-hydroxy-2-pyridon-1-yl)-6-nitro-2H-chromen
2,2-Dimethyl-4-(1H-4-hydroxy-2-pyridon-1-yl)-6-nitro-chroman-3-ol
2,2-Dimethyl-4-(1H-5-hydroxy-2-pyridon-1-yl)-6-nitro-2H-chromen
2,2-Dimethyl-4-(1H-5-hydroxy-2-pyridon-1-yl)-6-nitro-chroman-3-ol
2,2-Dimethyl-4-(1H-3-methoxy-2-pyridon-1-yl)-6-nitro-2H-chromen
2,2-Dimethyl-4-(1H-3-methoxy-2-pyridon-1-yl)-6-nitro-chroman-3-ol
2,2-Dimethyl-4-(1H-3-acetoxy-2-pyridon-1-yl)-6-nitro-2H-chromen
2,2-Dimethyl-4-(1H-3-acetoxy-2-pyridon-1-yl)-6-nitro-chroman-3-ol
2,2-Dimethyl-4-(1H-3-nitro-2-pyridon-1-yl)-6-nitro-2H-chromen
2,2-Dimethyl-4-(1H-3-nitro-2-pyridon-1-yl)-6-nitro-chroman-3-ol
2,2-Dimethyl-4-(1H-5-nitro-2-pyridon-1-yl)-6-nitro-2H-chromen
2,2-Dimethyl-4-(1H-5-nitro-2-pyridon-1-yl)-6-nitro-chroman-3-ol
2,2-Dimethyl-4-(1H-3-amino-2-pyridon-1-yl)-6-nitro-2H-chromen
2,2-Dimethyl-4-(1H-3-amino-2-pyridon-1-yl)-6-nitro-chroman-3-ol
2,2-Dimethyl-4-(1H-5-amino-2-pyridon-1-yl)-6-nitro-2H-chromen
2,2-Dimethyl-4-(1H-5-amino-2-pyridon-1-yl)-6-nitro-chroman-3-ol
2,2-Dimethyl-4-(1H-3-acetamido-2-pyridon-1-yl)-6-nitro-2H-chromen
2,2-Dimethyl-4-(1H-3-acetamido-2-pyridon-1-yl)-6-nitro-chroman-3-ol
2,2-Dimethyl-4-(1H-5-acetamido-2-pyridon-1-yl)-6-nitro-2H-chromen
2,2-Dimethyl-4-(1H-5-acetamido-2-pyridon-1-yl)-6-nitro-chroman-3-ol
2,2-Dimethyl-4-(1H-3-carboxy-2-pyridon-1-yl)-6-nitro-2H-chromen
2,2-Dimethyl-4-(1H-3-carboxy-2-pyridon-1-yl)-6-nitro-chroman-3-ol
2,2-Dimethyl-4-(1H-5-carboxy-2-pyridon-1-yl)-6-nitro-2H-chromen
2,2-Dimethyl-4-(1H-5-carboxy-2-pyridon-1-yl)-6-nitro-chroman-3-ol
2,2-Dimethyl-4-(1H-3,5-dichlor-2-pyridon-1-yl)-6-nitro-2H-chromen
2,2-Dimethyl-4-(1H-3,5-dichlor-2-pyridon-1-yl)-6-nitro-chroman-3-ol
2,2-Dimethyl-4-(1H-3,5-dibrom-2-pyridon-1-yl)-6-nitro-2H-chromen
2,2-Dimethyl-4-(1H-3,5-dibrom-2-pyridon-1-yl)-6-nitro-chroman-3-ol
2,2-Dimethyl-4-(1H-3-chlor-5-nitro-2-pyridon-1-yl)-6-nitro-2H-chromen
2,2-Dimethyl-4-(1H-3-chlor-5-nitro-2-pyridon-1-yl)-6-nitro-chroman-3-ol
2,2-Dimethyl-4-(1H-3-nitro-5-chlor-2-pyridon-1-yl)-6-nitro-2H-chromen
2,2-Dimethyl-4-(1H-3-nitro-5-chlor-2-pyridon-1-yl)-6-nitro-chroman-3-ol
2,2-Dimethyl-4-(1H-3-brom-5-nitro-2-pyridon-1-yl)-6-nitro-2H-chromen
2,2-Dimethyl-4-(1H-3-brom-5-nitro-2-pyridon-1-yl)-6-nitro-chroman-3-ol
2,2-Dimethyl-4-(1H-3-nitro-5-brom-2-pyridon-1-yl)-6-nitro-2H-chromen
2,2-Dimethyl-4-(1H-3-nitro-5-brom-2-pyridon-1-yl)-6-nitro-chroman-3-ol
2,2-Dimethyl-4-(1H-3,5-dinitro-2-pyridon-1-yl)-6-nitro-2H-chromen
2,2-Dimethyl-4-(1H-3,5-dinitro-2-pyridon-1-yl)-6-nitro-chroman-3-ol
2,2-Dimethyl-4-(1H-3-chlor-5-amino-2-pyridon-1-yl)-6-nitro-2H-chromen
2,2-Dimethyl-4-(1H-3-chlor-5-amino-2-pyridon-1-yl)-6-nitro-chroman-3-ol
2,2-Dimethyl-4-(1H-3-amino-5-chlor-2-pyridon-1-yl)-6-nitro-2H-chromen
2,2-Dimethyl-4-(1H-3-amino-5-chlor-2-pyridon-1-yl)-6-nitro-chroman-3-ol
2,2-Dimethyl-4-(1H-3-brom-5-amino-2-pyridon-1-yl)-6-nitro-2H-chromen
2,2-Dimethyl-4-(1H-3-brom-5-amino-2-pyridon-1-yl)-6-nitro-chroman-3-ol
2,2-Dimethyl-4-(1H-3-amino-5-brom-2-pyridon-1-yl)-6-nitro-2H-chromen
2,2-Dimethyl-4-(1H-3-amino-5-brom-2-pyridon-1-yl)-6-nitro-chroman-3-ol
2,2-Dimethyl-4-(1H-3-chlor-5-acetamido-2-pyridon-1-yl)-6-nitro-2H-chromen
2,2-Dimethyl-4-(1H-3-chlor-5-acetamido-2-pyridon-1-yl)-6-nitro-chroman-3-ol

2,2-Dimethyl-4-(1H-3-acetamido-5-chlor-2-pyridon-1-yl)-6-nitro-2H-chromen
2,2-Dimethyl-4-(1H-3-acetamido-5-chlor-2-pyridon-1-yl)-6-nitro-chroman-3-ol
2,2-Dimethyl-4-(1H-3-brom-5-acetamido-2-pyridon-1-yl)-6-nitro-2H-chromen
2,2-Dimethyl-4-(1H-3-brom-5-acetamido-2-pyridon-1-yl)-6-nitro-chroman-3-ol
2,2-Dimethyl-4-(1H-3-acetamido-5-brom-2-pyridon-1-yl)-6-nitro-2H-chromen
2,2-Dimethyl-4-(1H-3-acetamido-5-brom-2-pyridon-1-yl)-6-nitro-chroman-3-ol
2,2-Dimethyl-4-(1H-2-pyridon-1-yl)-6-acetyl-2H-chromen, F. 148-150°
2,2-Dimethyl-4-(1H-2-pyridon-1-yl)-6-acetyl-chroman-3-ol, F. 257-259°
2,2-Dimethyl-4-(1H-2-pyridon-1-yl)-6-methoxycarbonyl-2H-chromen, F. 126-127°
2,2-Dimethyl-4-(1H-2-pyridon-1-yl)-6-methoxycarbonyl-chroman-3-ol, F. 267-267,5°
2,2-Dimethyl-4-(1H-2-pyridon-1-yl)-6-ethoxycarbonyl-2H-chromen
2,2-Dimethyl-4-(1H-2-pyridon-1-yl)-6-ethoxycarbonyl-chroman-3-ol, F. 213°
2,2-Dimethyl-4-(1H-2-pyridon-1-yl)-6-fluor-2H-chromen
2,2-Dimethyl-4-(1H-2-pyridon-1-yl)-6-fluor-chroman-3-ol
2,2-Dimethyl-4-(1H-2-pyridon-1-yl)-6-chlor-2H-chromen
2,2-Dimethyl-4-(1H-2-pyridon-1-yl)-6-chlor-chroman-3-ol
2,2-Dimethyl-4-(1H-2-pyridon-1-yl)-6-trifluormethyl-2H-chromen
2,2-Dimethyl-4-(1H-2-pyridon-1-yl)-6-trifluormethyl-chroman-3-ol
2,2-Dimethyl-4-(1H-2-pyridon-1-yl)-6-acetamido-2H-chromen
2,2-Dimethyl-4-(1H-2-pyridon-1-yl)-6-acetamido-chroman-3-ol
2,2-Dimethyl-4-(1H-2-pyridon-1-yl)-6-carbamoyl-2H-chromen, F. 250-252°
2,2-Dimethyl-4-(1H-2-pyridon-1-yl)-6-carbamoyl-chroman-3-ol
2,2-Dimethyl-4-(1H-2-pyridon-1-yl)-6-thiocarbamoyl-2H-chromen
2,2-Dimethyl-4-(1H-2-pyridon-1-yl)-6-thiocarbamoyl-chroman-3-ol
2,2-Dimethyl-4-(1H-2-pyridon-1-yl)-7-cyan-2H-chromen
2,2-Dimethyl-4-(1H-2-pyridon-1-yl)-7-cyan-chroman-3-ol
2,2-Dimethyl-4-(1H-2-pyridon-1-yl)-6-acetamido-7-nitro-2H-chromen
2,2-Dimethyl-4-(1H-2-pyridon-1-yl)-6-acetamido-7-nitro-chroman-3-ol
2,2-Dimethyl-4-(1H-3-nitro-2-pyridon-1-yl)-6-methoxy-carbonyl-2H-chromen
2,2-Dimethyl-4-(1H-3-nitro-2-pyridon-1-yl)-6-methoxycarbonyl-chroman-3-ol
2,2-Tetramethylen-4-(1H-2-pyridon-1-yl)-6-cyan-2H-chromen, F. 181-183°
2,2-Tetramethylen-4-(1H-2-pyridon-1-yl)-6-cyan-chroman-3-ol, F. 227-230°
2,2-Pentamethylen-4-(1H-2-pyridon-1-yl)-6-cyan-2H-chromen, F. 202-204°
2,2-Pentamethylen-4-(1H-2-pyridon-1-yl)-6-cyan-chroman-3-ol, F. 240-242°
2,2-Dimethyl-4-(1H-2-pyridazinon-1-yl)-6-cyan-2H-chromen, F. 136-138°
2,2-Dimethyl-4-(1H-2-pyridazinon-1-yl)-6-cyan-chroman-3-ol, F. 216-218°
2,2-Dimethyl-4-(4,5-dihydro-1H-6-pyridazinon-1-yl)-6-cyan-2H-chromen
2,2-Dimethyl-4-(4,5-dihydro-1H-6-pyridazinon-1-yl)-6-cyan-chroman-3-ol, F. 163-164,5°
2,2-Dimethyl-4-(1H-3-hydroxy-6-pyridazinon-1-yl)-6-cyan-2H-chromen
2,2-Dimethyl-4-(1H-3-hydroxy-6-pyridazinon-1-yl)-6-cyan-chroman-3-ol, F. 254-256°
2,2-Dimethyl-4-(1H-3-ethoxycarbonyl-6-pyridazinon-1-yl)-6-cyan-2H-chromen
2,2-Dimethyl-4-(1H-3-ethoxycarbonyl-6-pyridazinon-1-yl)-6-cyan-chroman-3-ol, F. 259-260,5°
2,2-Dimethyl-4-(1H-2-pyrimidinon-1-yl)-6-cyan-2H-chromen
2,2-Dimethyl-4-(1H-2-pyrimidinon-1-yl)-6-cyan-chroman-3-ol, F. 250-252°
2,2-Dimethyl-4-(1H-4-hydroxy-2-pyrimidinon-1-yl)-6-cyan-2H-chromen, F. 278-279,5°
2,2-Dimethyl-4-(1H-4-hydroxy-2-pyrimidinon-1-yl)-6-cyan-chroman-3-ol, kein F. bis 300°
2,2-Dimethyl-4-(1H-6-pyrimidinon-1-yl)-6-cyan-2H-chromen
2,2-Dimethyl-4-(1H-6-pyrimidinon-1-yl)-6-cyan-chroman-3-ol, F. 207-208°
2,2-Dimethyl-4-(1H-4-hydroxy-6-pyrimidinon-1-yl)-6-cyan-2H-chromen
2,2-Dimethyl-4-(1H-4-hydroxy-6-pyrimidinon-1-yl)-6-cyan-chroman-3-ol, F. 235-237°
2,2-Dimethyl-4-(1H-2-pyrazinon-1-yl)-6-cyan-2H-chromen, F. 136-138°
2,2-Dimethyl-4-(1H-2-pyrazinon-1-yl)-6-cyan-chroman-3-ol, F. 255-257°.

Beispiel 2

Ein Gemisch von 20,1 g IIa, 9,5 g Pyridon und 150 ml Triethylamin wird 2 Std. auf 110 ° erhitzt, abgekühlt, eingedampft und dann wie üblich aufgearbeitet. Man erhält "B", daneben nur Spuren "A".

Analog erhält man aus den entsprechenden 3,4-Epoxychromanen:

2-Methyl-4-(1H-2-pyridon-1-yl)-2H-chromen
2-Methyl-4-(1H-2-pyridon-1-yl)-6-cyan-2H-chromen
2-Methyl-4-(1H-2-pyridon-1-yl)-6-nitro-2H-chromen
2-Methyl-2-ethyl-4-(1H-2-pyridon-1-yl)-6-cyan-2H-chromen
2,2-Diethyl-4-(1H-2-pyridon-1-yl)-6-cyan-2H-chromen
2,2-Trimethylen-4-(1H-2-pyridon-1-yl)-6-cyan-2H-chromen
2,2-Tetramethylen-4-(1H-2-pyridon-1-yl)-6-cyan-2H-chromen, F. 181-183°
2,2-Pentamethylen-4-(1H-2-pyridon-1-yl)-6-cyan-2H-chromen, F. 202-204°
2,2-Hexamethylen-4-(1H-2-pyridon-1-yl)-6-cyan-2H-chromen
2,2-Dimethyl-4-(1H-6-methyl-2-pyridon-1-yl)-6-cyan-2-chromen
2,2-Dimethyl-4-(1H-3-fluor-2-pyridon-1-yl)-6-cyan-2-chromen
2,2-Dimethyl-4-(1H-3,5-dijod-2-pyridon-1-yl)-6-cyan-2H-chromen
2,2-Dimethyl-4-(1H-5-hydroxy-2-pyridon-1-yl)-6-cyan-2H-chromen
2,2-Dimethyl-4-(1H-5-methoxy-2-pyridon-1-yl)-6-cyan-2H-chromen
2,2-Dimethyl-4-(1H-5-methoxycarbonyl-2-pyridon-1-yl)-6-cyan-2H-chromen
2,2-Dimethyl-4-(1H-2-pyridon-1-yl)-2H-chromen
2,2-Dimethyl-4-(1H-2-pyridon-1-yl)-6-methyl-2H-chromen
2,2-Dimethyl-4-(1H-2-pyridon-1-yl)-6-methoxy-2H-chromen
2,2-Dimethyl-4-(1H-2-pyridon-1-yl)-6-thioacetyl-2H-chromen
2,2-Dimethyl-4-(1H-2-pyridon-1-yl)-6-methoxy-thiocarbonyl-2H-chromen
2,2-Dimethyl-4-(1H-2-pyridon-1-yl)-6-thio(no)acetoxy-2H-chromen
2,2-Dimethyl-4-(1H-2-pyridon-1-yl)-6-hydroxymethyl-2H-chromen
2,2-Dimethyl-4-(1H-2-pyridon-1-yl)-6-dimethylamino-2H-chromen
2,2-Dimethyl-4-(1H-2-pyridon-1-yl)-6-brom-2H-chromen
2,2-Dimethyl-4-(1H-2-pyridon-1-yl)-6-jod-2H-chromen
2,2-Dimethyl-4-(1H-2-pyridon-1-yl)-6-methylsulfinyl-2H-chromen
2,2-Dimethyl-4-(1H-2-pyridon-1-yl)-6-methylsulfonyl-2H-chromen

Beispiel 3

Ein Gemisch von 2 g IIa und 1 g Pyridon wird 2 Std. auf 110 ° erhitzt, abgekühlt und der Rückstand wie üblich aufgearbeitet. Man erhält "B", daneben nur Spuren "A".

Beispiel 4

Ein Gemisch von 2 g IIa, 1,17 g Pyridon-Na-Salz und 30 ml Ethanol wird 3 Std. gekocht. Nach Aufarbeitung analog Beispiel 1 erhält man "A" und "B" im Mengenverhältnis ca. 3:2.

Beispiel 5

Eine Lösung von 2 g IIa und 1,17 g Pyridon-Na-Salz in 30 ml Ethanol wird 16 Std. bei 20 ° stehengelassen. Man erhält "B", daneben nur Spuren "A".

Beispiel 6

Eine Lösung von 2 g IIa und 1,17 g Pyridon-Na-Salz in 30 ml Ethanol und 2 ml Pyridin wird 1,5 Std. gekocht und abgekühlt; das ausgefallene "B" wird abfiltriert. Dabei entsteht "A" nur in Spuren.

Beispiel 7

Unter Rühren werden 0,4 g einer 60 %igen Öldispersion von NaH zu einer Lösung von 2,82 g trans-2,2-Dimethyl-3-brom-6-cyan-chroman-4-ol in 15 ml DMSO gegeben. Man rührt 1 Std., wobei intermediär 2,2-Dimethyl-3,4-epoxy-6-cyan-chroman entsteht. Man gibt 1,43 g 1H-2-Pyridon und weitere 0,5 g NaH-Dispersion hinzu und rührt 16 Std. bei 20 °. Nach Aufarbeitung analog Beispiel 1 erhält man "A" (F. 146-148 °) und "B" (F. 244 °).

Beispiel 8

Zu einer Lösung von 1 g "B" in 30 ml DMSO gibt man 96 mg 80 %iges NaH in Paraffinöl und läßt 16 Std. bei 20 ° stehen. Nach üblicher Aufarbeitung erhält man "A", F. 146-148 °.

Beispiel 9

Ein Gemisch von 2 g "B", 11,7 ml Ameisensäure und 3,3 ml Acetanhydrid wird 16 Std. bei 20° stehengelassen und anschließend 2 Std. auf 40-42° erwärmt. Nach Eindampfen und üblicher Aufarbeitung erhält man 2,2-Dimethyl-3-formyloxy-4-(1H-2-pyridon-1-yl)-6-cyan-chroman, F. 203,5-204°.

Analog erhält man aus den entsprechenden 3-Hydroxy-chromanen:

2,2-Dimethyl-3-formyloxy-4-(1H-2-pyridon-1-yl)-6-nitro-chroman, F. 188-193°
2,2-Dimethyl-3-formyloxy-4-(1H-4-hydroxy-2-pyridon-1-yl)-6-cyan-chroman
2,2-Dimethyl-3-formyloxy-4-(1H-3-hydroxy-6-pyridazinon-1-yl)-6-chroman

Beispiel 10

Ein Gemisch von 1 g "B" und 5 ml Acetanhydrid wird 1 Std. gekocht. Man kühlt ab, arbeitet wie üblich auf und erhält 2,2-Dimethyl-3-acetoxy-4-(1H-2-pyridon-1-yl)-6-cyan-chroman, F. 228-228,5°.

Beispiel 11

Man suspendiert 2,96 g "B" in 100 ml Wasser und tropft unter Rühren bei 10-20 ° 3,2 g Brom hinzu. Die Substanz löst sich, 2,2-Dimethyl-4-(1H-3,5-dibrom-2-pyridon-1-yl)-6-cyan-chroman-3-ol fällt aus und wird abfiltriert, F. 207-209°.

Beispiel 12

Man löst 2,78 g "A" in einem Gemisch von 10 ml konzentrierter Salpetersäure (68 %ig; D. 1,41) und 12 ml konzentrierter Schwefelsäure, rührt 3 Std. bei 20 °, gießt auf Eis, filtriert, wäscht mit Wasser und erhält ein Gemisch von 2,2-Dimethyl-4-(1H-3-und -5-nitro-pyridon-1-yl)-6-cyan-2H-chromen im Verhältnis von ca. 1:3, das chromatographisch getrennt werden kann.

Beispiel 13

Eine Lösung von 1 g 2,2-Dimethyl-4-(1H-3-nitro-2-pyridon-1-yl)-6-methoxycarbonyl-chroman-3-ol in 25 ml Methanol wird bei 20 ° und 1 bar an 0,5 g 5 %igem Pd-C bis zum Stillstand hydriert. Man filtriert, dampft ein und erhält 2,2-Dimethyl-4-(1H-3-amino-2-pyridon-1-yl)-6-methoxycarbonyl-chroman-3-ol.

Beispiel 14

Eine Lösung von 1 g 2,2-Dimethyl-4-(1H-3-amino-2-pyridon-1-yl)-6-cyan-2H-chromen in 15 ml HCOOH und 1 ml Pyridin wird 19 Std. gekocht und eingedampft. Nach üblicher Aufarbeitung erhält man 2,2-Dimethyl-4-(1H-3-formamido-2-pyridon-1-yl)-6-cyan-2H-chromen.

Beispiel 15

Ein Gemisch von 1 g 2,2-Dimethyl-4-(1H-5-amino-2-pyridon-1-yl)-6-cyan-2H-chromen, 10 ml Acetanhydrid und 10 ml Pyridin wird 16 Std. bei 20 ° stehengelassen. Man dampft ein, reinigt chromatographisch und erhält 2,2-Dimethyl-4-(1H-5-acetamido-2-pyridon-1-yl)-6-cyan-2H-chromen.

Beispiel 16

In eine siedende Lösung von 1 g "A" in 50 ml Methanol und 2 ml Wasser wird 14 Std. unter Rühren HCl eingeleitet. Man läßt erkalten und über Nacht stehen. Die ausgefallene 2,2-Dimethyl-4-(1H-2-pyridon-1-yl)-2H-chromen-6-carbonsäure wird abfiltriert, F. 281-284°.

Beispiel 17

Ein Gemisch von 2,78 g "A", 31 g $Na_3PO_4$ . 12 $H_2O$, 28 ml Pyridin, 28 ml Wasser, 67 ml Essigsäure und 25 g Raney-Ni (wasserfeucht) wird bei 20 ° 3 Std. gerührt. Nach Filtration arbeitet man wie üblich auf und erhält 2,2-Dimethyl-4-(1H-2-pyridon-1-yl)-6-formyl-2H-chromen, F. 160-162°.

Analog erhält man

2,2-Dimethyl-4-(1H-2-pyridon-1-yl)-6-formyl-chroman-3-ol, F. 210-214°
2,2-Dimethyl-4-(1H-2-pyridon-1-yl)-7-formyl-2H-chromen
2,2-Dimethyl-4-(1H-2-pyridon-1-yl)-7-formyl-chroman-3-ol
2,2-Dimethyl-4-(1H-4-hydroxy-2-pyridon-1-yl)-6-formyl-2H-chromen
2,2-Dimethyl-4-(1H-4-hydroxy-2-pyridon-1-yl)-6-formyl-chroman-3-ol
2,2-Dimethyl-4-(1H-3-hydroxy-6-pyridazinon-1-yl)-6-formyl-2H-chromen
2,2-Dimethyl-4-(1H-3-hydroxy-6-pyridazinon-1-yl)-6-formyl-chroman-3-ol.

Beispiel 18

Man löst 2,78 g "A" in 40 ml tert.-Butanol und gibt unter Rühren 5,6 g gepulvertes KOH hinzu. Nach 1 Std. Kochen und üblicher Aufarbeitung erhält man 2,2-Dimethyl-4-(1H-2-pyridon-1-yl)-6-carbamoyl-2H-chromen, F. 250-252°.

Analog erhält man

2,2-Dimethyl-4-(1H-2-pyridon-1-yl)-6-carbamoyl-chroman-3-ol
2,2-Dimethyl-4-(1H-2-pyridon-1-yl)-7-carbamoyl-2H-chromen
2,2-Dimethyl-4-(1H-2-pyridon-1-yl)-7-carbamoyl-chroman-3-ol
2,2-Dimethyl-4-(1H-4-hydroxy-2-pyridon-1-yl)-6-carbamoyl-2H-chromen
2,2-Dimethyl-4-(1H-4-hydroxy-2-pyridon-1-yl)-6-carbamoyl-chroman-3-ol
2,2-Dimethyl-4-(1H-3-hydroxy-6-pyridazinon-1-yl)-6-carbamoyl-2H-chromen
2,2-Dimethyl-4-(1H-3-hydroxy-6-pyridazinon-1-yl)-6-carbamoyl-chroman-3-ol.

Beispiel 19

In eine Lösung von 2,78 g "A" in einem Gemisch von 20 ml Pyridin und 10 ml Triethylamin leitet man 5 Std. lang bei 20 ° $H_2S$ ein, dampft ein, arbeitet wie üblich auf und erhält 2,2-Dimethyl-4-(1H-2-pyridon-1-yl)-6-thiocarbamoyl-2H-chromen, F. 254-257°.

Analog erhält man

2,2-Dimethyl-4-(1H-2-pyridon-1-yl)-6-thiocarbamoyl-chroman-3-ol, F. 228°
2,2-Dimethyl-4-(1H-2-pyridon-1-yl)-7-thiocarbamoyl-2H-chromen
2,2-Dimethyl-4-(1H-2-pyridon-1-yl)-7-thiocarbamoyl-chroman-3-ol
2,2-Dimethyl-4-(1H-4-hydroxy-2-pyridon-1-yl)-6-thiocarbamoyl-2H-chromen
2,2-Dimethyl-4-(1H-4-hydroxy-2-pyridon-1-yl)-6-thiocarbamoyl-chroman-3-ol
2,2-Dimethyl-4-(1H-3-hydroxy-6-pyridazinon-1-yl)-6-thiocarbamoyl-2H-chromen
2,2-Dimethyl-4-(1H-3-hydroxy-6-pyridazinon-1-yl)-6-thiocarbamoyl-chroman-3-ol.

Beispiel 20

Ein Gemisch von 296 mg "B", 808 mg Lawesson-Reagenz und 50 ml Toluol wird 1 Std. unter $N_2$ gekocht. Übliche Aufarbeitung gibt 2,2-Dimethyl-4-(1H-2-thiopyridon-1-yl)-6-cyan-chroman-3-ol.

Analog erhält man aus "A" das 2,2-Dimethyl-4-(1H-2-thiopyridon-1-yl)-6-cyan-2H-chromen.

Beispiel 21

Analog Beispiel 1 erhält man:

2,2-Dimethyl-4-(1H-4-methoxy-2-pyridon-1-yl)-6-cyan-2H-chromen, F. 92-95°
2,2-Dimethyl-4-(1H-4-methoxy-2-pyridon-1-yl)-6-cyan-chroman-3-ol, F. 228-230°
2,2-Dimethyl-4-(1H-4-ethoxy-2-pyridon-1-yl)-6-cyan-2H-chromen, F. 102-104°
2,2-Dimethyl-4-(1H-4-ethoxy-2-pyridon-1-yl)-6-cyan-chroman-3-ol, F. 210-212°
2,2-Dimethyl-4-(1H-4-acetoxy-2-pyridon-1-yl)-6-cyan-2H-chromen, F. 170-172°
2,2-Dimethyl-4-(1H-4-acetoxy-2-pyridon-1-yl)-6-cyan-chroman-3-ol
2,2-Dimethyl-4-(1H-2-pyridon-1-yl)-6-cyan-8-nitro-2H-chromen, F. 149-151°
2,2-Dimethyl-4-(1H-2-pyridon-1-yl)-6-cyan-8-nitro-chroman-3-ol
2,2-Dimethyl-4-(1H-2-pyridon-1-yl)-6-hydroxymethyl-2H-chromen
2,2-Dimethyl-4-(1H-2-pyridon-1-yl)-6-hydroxymethyl-chroman-3-ol, F. 170-172°
2,2-Tetramethylen-4-(1H-2-pyridon-1-yl)-6-nitro-2H-chromen, F. 229-230°
2,2-Tetramethylen-4-(1H-2-pyridon-1-yl)-6-nitro-chroman-3-ol, F. 249°
2,2-Pentamethylen-4-(1H-2-pyridon-1-yl)-6-nitro-2H-chromen, F. 210-212°
2,2-Pentamethylen-4-(1H-2-pyridon-1-yl)-6-nitro-chroman-3-ol, F. 247°

2,2-Dimethyl-4-(1H-3-methoxy-6-pyridazinon-1-yl)-6-cyan-2H-chromen
2,2-Dimethyl-4-(1H-3-methoxy-6-pyridazinon-1-yl)-6-cyan-chroman-3-ol, F. 165-167°

2,2-Dimethyl-4-(1H-4-hydroxy-2-pyridon-1-yl)-6-acetyl-2H-chromen
2,2-Dimethyl-4-(1H-4-hydroxy-2-pyridon-1-yl)-6-acetyl-chroman-3-ol
2,2-Dimethyl-4-(1H-4-hydroxy-2-pyridon-1-yl)-6-methoxy-carbonyl-2H-chromen
2,2-Dimethyl-4-(1H-4-hydroxy-2-pyridon-1-yl)-6-methoxycarbonyl-chroman-3-ol
2,2-Dimethyl-4-(1H-4-hydroxy-2-pyridon-1-yl)-6-ethoxycarbonyl-2H-chromen
2,2-Dimethyl-4-(1H-4-hydroxy-2-pyridon-1-yl)-6-ethoxycarbonyl-chroman-3-ol

2,2-Dimethyl-4-(1H-3-hydroxy-6-pyridazinon-1-yl)-6-acetyl-2H-chromen
2,2-Dimethyl-4-(1H-3-hydroxy-6-pyridazinon-1-yl)-6-acetyl-chroman-3-ol
2,2-Dimethyl-4-(1H-3-hydroxy-6-pyridazinon-1-yl)-6-methoxycarbonyl-2H-chromen
2,2-Dimethyl-4-(1H-3-hydroxy-6-pyridazinon-1-yl)-6-methoxycarbonyl-chroman-3-ol
2,2-Dimethyl-4-(1H-3-hydroxy-6-pyridazinon-1-yl)-6-ethoxycarbonyl-2H-chromen
2,2-Dimethyl-4-(1H-3-hydroxy-6-pyridazinon-1-yl)-6-ethoxycarbonyl-chroman-3-ol
2,2-Dimethyl-4-(1H-3-hydroxy-6-pyridazinon-1-yl)-6-nitro-2H-chromen
2,2-Dimethyl-4-(1H-3-hydroxy-6-pyridazinon-1-yl)-6-nitro-chroman-3-ol.

Die nachstehenden Beispiele betreffen pharmazeutische Zubereitungen, die Verbindungen der Formel I oder ihre physiologisch unbedenklichen Salze enthalten:

Beispiel A Tabletten

Ein Gemisch von 1 kg 2,2-Dimethyl-4-(1H-2-pyridon,-1-yl)-6-cyan-2H-chromen, 4 kg Lactose, 1,2 kg Kartoffelstärke, 0,2 g Talk und 0,1 kg Magnesiumstearat wird in üblicher Weise zu Tabletten gepreßt, derart, daß jede Tablette 10 mg Wirkstoff enthält.

Beispiel B Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C Kapseln

Man füllt 1 kg 2,2-Dimethyl-4-(1H-2-pyridon-1-yl)-6-cyan-chroman-3-ol in üblicher Weise in Hartgelatinekapseln, so daß jede Kapsel 20 mg Wirkstoff enthält.

Beispiel D Ampullen

Eine Lösung von 1 kg 2,2-Dimethyl-4-(1H-2-pyridon-1-yl)-6-nitro-2H-chromen in einem Gemisch von 20 l 1,2-Propandiol und 10 l zweifach destilliertem Wasser wird steril filtriert, in Ampullen abgefüllt, unter sterilen Bedingungen lyophilisiert und steril verschlossen. Jede Ampulle enthält 5 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

**Ansprüche**

1. Chromanderivate der Formel I

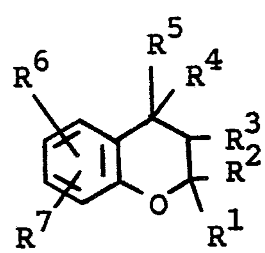

I

worin

$R^1$ A,

$R^2$ H oder A,

$R^1$ und $R^2$ zusammen auch Alkylen mit 3-6 C-Atomen,

$R^3$ OH oder OAc,

$R^4$ H,

$R^3$ und $R^4$ zusammen auch eine Bindung,

$R^5$ einen unsubstituierten oder ein-oder zweifach durch A, F, Cl, Br, J, OH, OA, OAc, $NO_2$, $NH_2$, AcNH, HOOC und/oder AOOC substituierten 1H-2-Pyridon-1-yl-, 1H-6-Pyridazinon-1-yl, 1H-2-Pyrimidinon-1-yl, 1H-6-Pyrimidinon-1-yl, 1H-2-Pyrazinon-1-yl oder 1H-2-Thiopyridon-1-yl-rest, wobei diese Reste auch partiell

hydriert sein können,

$R^6$ und $R^7$ jeweils H, A, HO, AO, CHO, ACO, ACS, HOOC, AOOC, AO-CS, ACOO, A-CS-O, Hydroxyalkyl mit 1-6 C-Atomen, Mercaptoalkyl mit 1-6 C-Atomen, $NO_2$, $NH_2$, NHA, $NA_2$, CN, F, Cl, Br, J, $CF_3$, ASO, $ASO_2$, AO-SO, AO-$SO_2$, AcNH, AO-CO-NH, $H_2NSO$, HANSO, $A_2NSO$, $H_2NSO_2$, $HANSO_2$, $A_2NSO_2$, $H_2NCO$, HANCO, $A_2NCO$, $H_2NCS$, HANCS, $A_2NCS$, ASONH, $ASO_2NH$, AOSONH, $AOSO_2NH$, ACO-alkyl, Nitro-alkyl, Cyan-alkyl, A-C(=NOH) oder A-C(=$NNH_2$),

A Alkyl mit 1-6 C-Atomen,

alkyl Alkylen mit 1-6 C-Atomen und

Ac Alkanoyl mit 1-8 C-Atomen oder Aroyl mit 7-11 C-Atomen

bedeuten,

sowie deren Salze.

2. a) 2,2-Dimethyl-4-(1H-2-pyridon-1-yl)-6-cyan-2H-chromen;
b) 2,2-Dimethyl-4-(1H-2-pyridon-1-yl)-6-cyan-chroman-3-ol;
c) 2,2-Dimethyl-4-(1H-2-pyridon-1-yl)-6-nitro-2H-chromen;
d) 2,2-Dimethyl-4-(1H-2-pyridon-1-yl)-6-nitro-chroman-3-ol;
e) 2,2-Dimethyl-4-(1H-4-hydroxy-2-pyridon-1-yl)-6-cyanchroman-3-ol;
f) 2,2-Dimethyl-4-(1H-2-pyrazinon-1-yl)-6-cyan-2H-chromen;
g) 2,2-Dimethyl-4-(1H-2-pyrazinon-1-yl)-6-cyan-chroman-3-ol.

3. Verfahren zur Herstellung von Chromanderivaten der Formel I, dadurch gekennzeichent, daß man ein 3,4-Epoxychroman der Formel II

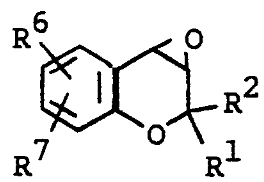

II

worin

$R^1$, $R^2$, $R^6$ und $R^7$ die bei Formel I angegebene Bedeutung haben,

mit einer Verbindung der Formel III

$R^5$-H III

worin $R^5$ die bei Formel I angegebene Bedeutung hat oder mit einem ihrer reaktionsfähigen Derivate umsetzt

und/oder daß man eine Verbindung der Formel I, worin $R^3$ OH und $R^4$ H bedeutet, dehydratisiert und/oder daß man in einer Verbindung der Formel I einen oder mehrere der Reste $R^3$, $R^5$, $R^6$ und/oder $R^7$ in andere Reste $R^3$, $R^5$, $R^6$ und/oder $R^7$ umwandelt und/oder daß man eine basische Verbindung der Formel I durch Behandeln mit einer Säure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I zur Bekämpfung von Krankheiten.

7. Verwendung von Verbindungen der Formel I zur Herstellung eines Arzneimittels.

8. Verwendung von Verbindungen der Formel I bei der Bekämpfung von Krankheiten.